# EUROPÄISCHE PATENTSCHRIFT

(11) **EP 0 807 127 B1**
(45) Veröffentlichungstag und Bekanntmachung des Hinweises auf die Patenterteilung: **08.12.1999**
(21) Anmeldenummer: 96901769.8
(22) Anmeldetag: 26.01.1996
(51) Int. Cl.: C08B 37/08

(54) **VERFAHREN ZUR HERSTELLUNG VON NIEDRIGVISKOSEN KATIONISCHEN BIOPOLYMEREN**
METHOD OF PREPARING LOW-VISCOSITY CATIONIC BIOPOLYMERS
PROCEDE DE PRODUCTION DE BIOPOLYMERES CATIONIQUES DE FAIBLE VISCOSITE

(30) Priorität: 03.02.1995 DE 19503465
(43) Veröffentlichungstag der Anmeldung: 19.11.1997
(73) Patentinhaber: Cognis Deutschland GmbH, 40589 Düsseldorf (DE)
(72) Erfinder: TANABE, Bettina, D-41352 Korschenbroich (DE); WACHTER, Rolf, D-40595 Düsseldorf (DE)
(86) Internationale Anmeldenummer: EP9600318
(87) Internationale Veröffentlichungsnummer: WO9623817

(56) Entgegenhaltungen:
- WO-A-91/05808
- FR-A- 2 701 266
- GB-A- 458 839
- PATENT ABSTRACTS OF JAPAN vol. 13, no. 174 (C-589), 25.April 1989 & JP,A,01 005553 (SHINTO PAINT CO LTD), 10.Januar 1989,
- JOURNAL OF APPLIED POLYMER SCIENCE, Bd. 33, Nr. 1, 1.Januar 1987, NEW YORK US, Seiten 177-189, XP002004402 HENRYK STRUCZCZYK: "Microcrystalline Chitosan"
- DATABASE WPI Week 9443, Derwent Publications Ltd., London, GB; AN 94-348281, XP002004403 & SU,A,1 821 471 (FAR E FISHING IND TECH INST) 15 Juni 1993
- Proceedings of the Internat. Conference on Chitin/Chitosan held in Boston, May 1978, L.J.Filar et al."Bulk and Solution Properties of Chitosan", pages 169-184

## Beschreibung

Die Erfindung betrifft ein Verfahren zur Herstellung von niedrigviskosen kationischen Biopolymeren durch Nachbehandlung von bekannten Deacetylierungsprodukten des Chitins sowie die Verwendung der Biopolymeren zur Herstellung von kosmetischen und/oder pharmazeutischen Mitteln.

### Stand der Technik

Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den - idealisierten - Monomerbaustein (I) enthalten. Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln, aber auch als Verdicker in Tensidgemischen eingesetzt. 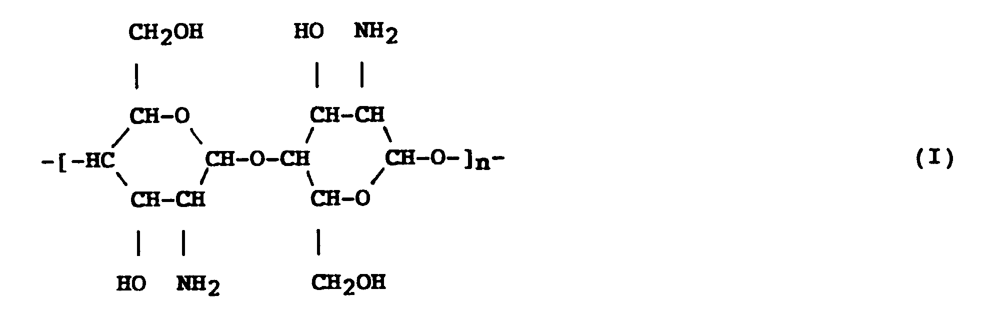 Übersichten zu diesem Thema sind beispielsweise von B.Gesslein et al. in **HAPPI 27, 57 (1990)**, O.Skaugrud in **Drug Cola. Ind. 148, 24 (1991)** und E.Onsoyen et al. in **Seifen**-Öle-Fette-**Wachse 117, 633 (1991)** erschienen.

Zur Herstellung der Chitosane geht man von Chitin, vorzugsweise den Schalenresten von Krustentieren aus, die als billige Rohstoffe in großen Mengen zur Verfügung stehen. Das Chitin wird dabei üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren zur Herstellung von - mikrokristallinem - Chitosan sind beispielsweise in der **WO 91/05808** (Firextra Oy) und der **EP-B1 0382150** (Hoechst) beschrieben.

Kationische Biopolymere der genannten Art weisen jedoch mehrere Nachteile auf: Sie sind schwerlöslich, trüben aus, weisen in wäßrigen organischen Säuren eine gelförmige, häufig zu hohe Viskosität auf und sind anfällig gegenüber mikrobieller Kontamination.

Die komplexe Aufgabe der Erfindung hat demnach darin bestanden, ein verbessertes Verfahren zur Herstellung von kationischen Biopolymeren zur Verfügung zu stellen, das frei von den geschilderten Nachteilen sind.

### Beschreibung der Erfindung

Gegenstand der Erfindung ist ein Verfahren zur Herstellung von niedrigviskosen kationischen Biopolymeren mit einem durchschnittlichen Molekulargewicht von etwa 10.000 bis etwa 2.000.000 und vorzugsweise einem Aschegehalt von weniger als 0,3 Gew.-%, bei dem man
(a) frische Krustentierschalen mit verdünnter wäßriger Mineralsäure behandelt,
(b) das resultierende demineralisierte erste Zwischenprodukt mit wäßriger Alkalihydroxidlösung behandelt,
(c) das resultierende geringfügig deproteinierte zweite Zwischenprodukt erneut mit verdünnter wäßriger Mineralsäure behandelt,
(d) das resultierende decalcifizierte dritte Zwischenprodukt schließlich mit konzentrierter wäßriger Alkalilauge behandelt und dabei bis zu einem Gehalt von 0,15 bis 0,8 und insbesondere 0,2 bis 0,25 Mol Acetamid pro Mol Monomereinheit deacetyliert und
(e) das deacetylierte vierte Zwischenprodukt einer Nachbehandlung bei einer Temperatur im Bereich von 50 bis 100 °C und gegebenenfalls einem Druck von 1,5 bis 3,0 bar unterwirft.
Überraschenderweise wurde gefunden, daß sich kationische Biopolymere erhalten lassen, die in organischen Säuren gelöst eine vorteilhaft niedrige Viskosität aufweisen, wenn man an sich bekannte Deacetylierungsprodukte von seetierischem Chitin einer Temperaturnachbehandlung, gegebenenfalls unter erhöhtem Druck unterwirft.

### Einsatzstoffe

Als Einsatzstoffe kommen Schalen von Krustentieren, vorzugsweise Krebs-, Krabben-, Shrimps- oder Krillschalen in Frage. Im Hinblick auf die Kontaminierung durch Endotoxine und die antimikrobielle Stabilisierung der Endprodukte hat es sich als vorteilhaft erwiesen, fangfrische Rohstoffe einzusetzen. Dies kann in der Praxis z.B. bedeuten, daß frisch gefangene Krabben noch an Bord des Schiffes von ihren Schalen befreit und diese bis zu ihrer Verarbeitung tiefgefroren werden. Es ist natürlich ebenso möglich, den gesamten Fang einzufrieren und an Land weiterzuverarbeiten.

### Demineralisierung

Die Demineralisierung ist als erster Schritt des erfindungsgemäßen Verfahrens besonders wichtig, da auf diesem Wege Endotoxine entfernt, die Deproteinierung wesentlich erleichtert und schließlich die Grundlagen für die Herstellung von besonders aschearmen Biopolymeren gelegt werden. Die Demineralisierung wird durch Behandlung der Schalen mit wäßrigen Mineralsäuren, vorzugsweise verdünnter Salzsäure bei einer Temperatur im Bereich von 15 bis 25, vorzugsweise etwa 20°C und einem pH-Wert von 0,3 bis 0,7, vorzugsweise etwa 0,5 durchgeführt.

### Deproteinierung

Die Deproteinierung wird durch Behandeln der demineralisierten Zwischenprodukte mit wäßrigen Alkalihydroxidlösungen, vorzugsweise Natriumhydroxidlösung erreicht. Vorzugsweise führt man diesen Schritt bei einer Temperatur im Bereich von 50 bis 95, insbesondere 70 bis 80°C und einem pH-Wert von 12 bis 14 durch.

### Decalcifizierung

Die Decalcifizierung gleicht in der Durchführung dem Demineralisierungsschritt. Auch hier wird das nunmehr deproteinierte Zwischenprodukt mit wäßrigen Mineralsäuren bei einer Temperatur im Bereich von 15 bis 25, vorzugsweie etwa 20°C und einem pH-Wert von 0,3 bis 0,7, vorzugsweise etwa 0,5 behandelt. Es hat sich als vorteilhaft erwiesen, das decalcifizierte Zwischenprodukt vor der nachfolgenden Deacetylierung bis auf einen Restwassergehalt von 5 bis 25 Gew.-% - bezogen auf das nichtentwässerte Produkt - zu trocknen.

### Deacetylierung

Die Deacetylierung wird unter Verwendung von konzentrierten Basen, beispielsweise konzentrierter Natron- oder Kalilauge wiederum bei einer Temperatur im Bereich von 50 bis 95, insbesondere 70 bis 98°C und einem pH-Wert von 12 bis 14 durchgeführt. Der Abbau wird soweit durchgeführt, daß ein Biopolymer erhalten wird, das einen Gehalt von 0,15 bis 0,25, vorzugsweise 0,16 bis 0,2 Mol Acetamid pro Mol Monomerbaustein resultiert. Der Deacetylierungsgrad kann im wesentlichen durch die Reaktionszeit eingestellt werden, die üblicherweise bei 1 bis 10 und vorzugsweise 2 bis 5 h liegt. Es hat sich ferner als vorteilhaft erwiesen, die Zwischenprodukte vor jedem neuen Verfahrensschritt neutral zu waschen.

### Temperatur/Drucknachbehandlung

Die nach den Verfahrensschritten (a) bis (d) erhältlichen kationischen Biopolymeren stellen Gele dar, die als 2 Gew.-% Lösungen z. B. in Glycol- oder Essigsäure Viskositäten nach Brookfield im Bereich von 20.000 bis 30.000 mPa*s aufweisen. Durch eine Nachbehandlung bei erhöhten Temperaturen und gegebenenfalls bei erhöhtem Druck kann die Viskosität soweit herabgesetzt werden, daß niedrigviskose Produkte mit einer Viskosität nach Brookfield im Bereich von 100 bis 2000 mPa^{∗}s resultieren. Typischerweise findet die Temperaturnachbehandlung beispielsweise in einem Rührkessel bei Temperaturen im Bereich von 50 bis 100 und vorzugsweise 80 bis 95°C statt. Für die Temperaturnachbehandlung unter Druck empfiehlt sich die Verwendung eines Autoklaven. Bei der Autoklavierung stellt sich in Abhängigkeit von der Temperatur in der Regel ein autogener Druck im Bereich von 1,5 bis 3,0 und vorzugsweise etwa 2 bar ein. Neben den kationischen Biopolymeren können auch deren Lösungen in organischen Säuren wie Essigoder Glycolsäure autoklaviert werden.

### Gewerbliche Anwendbarkeit

Die nach dem erfindungsgemäßen Verfahren erhältlichen kationischen Biopolymeren lösen sich in organischen Säuren wie z.B. Essigsäure oder Glycolsäure klar und zeichnen sich beispielsweise als 2 Gew.-%ige Lösungen durch eine Viskosität nach Brookfield aus, die im Bereich von 100 bis 2000 und vorzugsweise von 200 bis 500 mPa*s liegt. Sie eignen sich daher zur Herstellung von kosmetischen Produkten wie beispielsweise Mitteln zur Haut- und Haarpflege und insbesondere Mitteln, die in Form von Sprays formuliert werden. Wird die Temperaturnachbehandlung im Autoklaven unter Druck durchgeführt, werden sterile Produkte erhalten, die sich in ausgezeichneter Weise auch für die Herstellung von pharmazeutischen Mitteln eignen.

Die folgenden Beispiele sollen den Gegenstand der Erfindung näher erläutern, ohne ihn darauf einzuschränken.

### Beispiele

### I. Herstellbeispiele

### Vergleichsbeispiel V1:

(a) 1000 g frisch ausgelöste Krabbenschalen (Aschegehalt 31,8 Gew.-% bezogen auf das Trockengewicht) wurden getrocknet und über einen Zeitraum von 12 h bei 18°C mit 3000 ml verdünnter, 1 molarer Salzsäure bei einem pH-Wert von 0,5 behandelt. Anschließend wurde das demineralisierte Produkt neutral gewaschen; der Aschegehalt - bezogen auf Trockengewicht - betrug 0,79 Gew.-%.
(b) Das demineralisierte Produkt aus (a) wurden mit 5 Gew.-%iger Natriumhydroxidlösung im Gewichtsverhältnis 1 : 3 versetzt und 2 h bei 70°C gerührt. Anschließend wurde das deproteinierte Produkt 18 h bei 30°C gehalten. Das resultierende Material wurde wieder neutral gewaschen; der Aschegehalt - bezogen auf Trockengewicht - betrug 0,6 Gew.-%.
(c) Das deproteinierte Produkt aus (b) wurde wiederum im Gewichtsverhältnis 1 : 3 mit 1 molarer Salzsäure versetzt. Der Ansatz wurde bei 18°C etwa 1,5 h leicht gerührt und anschließend neutral gewaschen. Das decalcifizierte, leicht rosa gefärbte Produkt wies einen Aschegehalt - bezogen auf Trockengewicht - von 0,08 Gew.-% auf und wurde vor der Weiterverabeitung durch Abpressen weitgehend von anhaftendem Wasser befreit.
(d) Das decalcifizierte Produkt aus (c) wurde im Gewichtsverhältnis 1 : 4 mit 65 Gew.-%iger wäßriger Natriumhydroxidlösung versetzt und 4 h bei 95°C gehalten. Danach wurde das resultierende Chitosan mit Wasser neutral gewaschen, gefriergetrocknet und zermahlen. Es wurde ein blaß rosa gefärbtes Pulver erhalten, das sich in 1 Gew.-%iger Essigsäure auflöste. Der Acetylierungsgrad betrug nach ¹H-NMR-Untersuchung 0,17 Mol Acetamid/Mol Monomerbaustein. Die Ergebnisse der Viskositätsmessungen sind in Tabelle 1 zusammengefaßt.

### Beispiel 1:

100 g des 1 Gew.-%ig in Glycolsäure gelösten Gels des kationischen Biopolymers aus V1 wurden in einen Dreihalskolben überführt und über einen Zeitraum von 24 h bei einer erhöhten Temperatur von 90°C nachbehandelt. Die Ergebnisse der Viskositätsmessungen sind in Tabelle 1 zusammengefaßt.

### Beispiel 2:

100 g des kationischen Biopolymers aus V1 wurden in einen Autoklaven überführt und über einen Zeitraum von 20 min bei einer erhöhten Temperatur von 120°C und einem autogenen Druck von 2 bar nachbehandelt. Die Ergebnisse der Viskositätsmessungen sind in Tabelle 1 zusammengefaßt.

### II. Viskositätsmessungen

Die Bestimmung der Viskosität erfolgte in einem Brookfield RVT-Viskosimeter bei 20°C (Spindel 5, 10 UpM).

**Tabelle 1**

| Viskositäten der 2 Gew.-%igen Gele | |
|---|---|
| **Bsp.** | **Viskosität** **mPa*s** |
| 1 | 200 |
| 2 | 1.500 |
| V1 | 25.000 |

## Patentansprüche

1. Verfahren zur Herstellung von niedrigviskosen kationischen Biopolymeren mit einem durchschnittlichen Molekulargewicht von etwa 10.000 bis etwa 2.000.000, bei dem man
(a) frische Krustentierschalen mit verdünnter wäßriger Mineralsäure behandelt,
(b) das resultierende demineralisierte erste Zwischenprodukt mit wäßriger Alkalihydroxidlösung behandelt,
(c) das resultierende geringfügig deproteinierte zweite Zwischenprodukt erneut mit verdünnter wäßriger Mineralsäure behandelt,
(d) das resultierende decalcifizierte dritte Zwischenprodukt schließlich mit konzentrierter wäßriger Alkalilauge behandelt und dabei bis zu einem Gehalt von 0,15 bis 0,8 Mol Acetamid pro Mol Monomereinheit deacetyliert und
(e) das deacetylierte vierte Zwischenprodukt einer Nachbehandlung bei einer Temperatur im Bereich von 50 bis 100°C und gegebenenfalls einem Druck von 1,5 bis 3 bar unterwirft.

2. Verfahren nach Anspruch 1, **dadurch gekennzeichnet,** daß man als Mineralsäure verdünnte Salzsäure einsetzt.

3. Verfahren nach den Ansprüchen 1 und 2, **dadurch gekennzeichnet,** daß man als Alkalihydroxidlösung Natriumhydroxidlösung einsetzt.

4. Verfahren nach den Ansprüchen 1 bis 3, **dadurch gekennzeichnet,** daß man die Schritte (a) und (c) bei einer Temperatur im Bereich von 15 bis 25°C und einem pH-Wert von 0,3 bis 0,7 durchführt.

5. Verfahren nach den Ansprüchen 1 bis 4, **dadurch gekennzeichnet,** daß man die Schritte (b) und (d) bei einer Temperatur im Bereich von 50 bis 95°C und einem pH-Wert von 12 bis 14 durchführt.

6. Verfahren nach den Ansprüchen 1 bis 5, **dadurch gekennzeichnet,** daß man das decalcifizierte Zwischenprodukt aus Schritt (c) vor der Deacetylierung bis auf einen Restwassergehalt von 5 bis 25 Gew.-% trocknet.

7. Verfahren nach den Ansprüchen 1 bis 6, **dadurch gekennzeichnet,** daß man die Zwischenprodukte vor jedem neuen Verfahrensschritt neutral wäscht.

8. Verwendung der kationischen Biopolymeren nach dem Verfahren nach den Ansprüchen 1 bis 7 zur Herstellung von kosmetischen und/oder pharmazeutischen Mitteln.

## Claims

1. A process for the production of low-viscosity cationic biopolymers with an average molecular weight of about 10,000 to about 2,000,000, in which
(a) fresh crustacean shells are treated with dilute aqueous mineral acid,
(b) the resulting demineralized first intermediate product is treated with aqueous alkali metal hydroxide solution,
(c) the resulting lightly deproteinized second intermediate product is retreated with dilute aqueous mineral acid,
(d) finally, the resulting decalcified third intermediate product is treated with concentrated aqueous alkali liquor and, in the process, is deacetylated to an acetamide content of 0.15 to 0.8 mol per mol of monomer unit and
(e) the deacetylated fourth intermediate product is aftertreated at a temperature of 50 to 100°C and optionally under a pressure of 1.5 to 3 bar.

2. A process as claimed in claim 1, characterized in that dilute hydrochloric acid is used as the mineral acid.

3. A process as claimed in claims 1 and 2, characterized in that sodium hydroxide solution is used as the alkali metal hydroxide solution.

4. A process as claimed in claims 1 to 3, characterized in that steps (a) and (c) are carried out at a temperature of 15 to 25°C and at a pH value of 0.3 to 0.7.

5. A process as claimed in claims 1 to 4, characterized in that steps (b) and (d) are carried out at a temperature of 50 to 95°C and at a pH value of 12 to 14.

6. A process as claimed in claims 1 to 5, characterized in that the decalcified intermediate product from step (c) is dried to a residual water content of 5 to 25% by weight before the deacetylation step.

7. A process as claimed in claims 1 to 6, characterized in that the intermediate products are washed to neutrality before each new step.

8. Use of the cationic biopolymers according to the claims 1 to 7 for the production of cosmetic and/or pharmazeutical products.

## Revendications

1. Procédé de production de biopolymères cationiques de faible viscosité présentant un poids moléculaire moyen d'environ 10.000 à environ 2.000.000, dans lequel on
(a) traite des écailles de crustacés fraîches avec de l'acide minéral aqueux dilué,
(b) traite le premier produit intermédiaire déminéralisé obtenu avec une solution aqueuse d'hydroxyde de métal alcalin,
(c) traite à nouveau le second produit intermédiaire légèrement déprotéinisé obtenu avec de l'acide minéral aqueux dilué,
(d) traite enfin le troisième produit intermédiaire décalcifié obtenu avec de la lessive alcaline aqueuse concentrée, en le désacétylant ainsi jusqu'à une teneur de 0,15 à 0,8 mol d'acétamide par mol d'unité monomère et on
(e) soumet le quatrième produit intermédiaire désacétylé à un traitement postérieur à une température située dans la plage de 50 à 100 °C et le cas échéant, à une pression de 1,5 à 3,0 bars.

2. Procédé selon la revendication 1, **caractérisé en ce que** l'on met en oeuvre comme acide minéral, de l'acide chlorhydrique dilué.

3. Procédé selon les revendications 1 et 2, **caractérisé en ce que** l'on met en oeuvre comme solution d'hydroxyde de métal alcalin, une solution d'hydroxyde de sodium.

4. Procédé selon les revendications 1 à 3, **caractérisé en ce que** l'on opère les étapes (a) et (c) à une température comprise dans l'intervalle de 15 à 25 °C et à un pH de 0,3 à 0,7.

5. Procédé selon les revendications 1 à 4, **caractérisé en ce que** l'on opère les étapes (b) et (d) à une température comprise dans l'intervalle de 50 à 95 °C et à un pH de 12 à 14.

6. Procédé selon les revendications 1 à 5, **caractérisé en ce que** l'on sèche le produit intermédiaire décalcifié de l'étape (c) avant la désacétylation, jusqu'à une teneur résiduelle en eau de 5 à 25 % en poids.

7. Procédé selon les revendications 1 à 6, **caractérisé en ce que** l'on lave les produits intermédiaires à neutralité avant chaque nouvelle étape du procédé.

8. Utilisation des biopolymères cationiques, produits selon le procédé conforme aux revendications 1 à 7, pour la fabrication de produits cosmétiques et/ou pharmaceutiques.
